# EUROPÄISCHE PATENTSCHRIFT

(11) **EP 0 398 277 B1**
(45) Veröffentlichungstag und Bekanntmachung des Hinweises auf die Patenterteilung: **22.12.1993**
(21) Anmeldenummer: 90109198.3
(22) Anmeldetag: 16.05.1990
(51) Int. Cl.: G01N 33/18

(54) **Vorrichtung zur Bestimmung des biochemischen Sauerstoffbedarfs und der Toxizität von insbesondere Abwässern**
Apparatus for determining the biochemical oxygen demand and the toxicity particularly of waste water
Appareil pour déterminer le besoin biochimique en oxygène et la toxicité d'eaux usées en particulier

(30) Priorität: 18.05.1989 DE 3916106
(43) Veröffentlichungstag der Anmeldung: 22.11.1990
(73) Patentinhaber: Grzesny, Rolf, Dipl.-Ing., D-92332 Berching (DE); Beckmann, Hans-Jürgen, Dr., D-44143 Dortmund (DE); Bergmann, Thomas, Dipl.-Kfm., D-49082 Osnabrück (DE)
(72) Erfinder: Grzesny, Rolf, Dipl.-Ing., D-92332 Berching (DE); Beckmann, Hans-Jürgen, Dr., D-44143 Dortmund (DE); Bergmann, Thomas, Dipl.-Kfm., D-49082 Osnabrück (DE)
(74) Vertreter: Busse & Busse Patentanwälte

(56) Entgegenhaltungen:
- DE-A- 2 307 339
- US-A- 3 772 176
- ISA TRANSACTIONS, Band 9, Nr. 1, 1970; A.I. MYTELKA etal., Seiten 17-21#

## Beschreibung

Die Erfindung bezieht sich auf eine Vorrichtung zur Bestimmung des biochemischen Sauerstoffbedarfs und der Toxizität von insbesondere Abwässern in einer Ausbildung gemäß dem Oberbegriff des Anspruchs 1.

Bei einer vollständigen Beurteilung eines Abwassers bzw. der Abwasserqualität spielt die Bewertung des biologisch abbaubaren Anteils und der Toxizität eine entscheidende Rolle. Hierbei wird herkömmlicherweise der biochemische Sauerstoffbedarf (BSB) ermittelt, der von Mikroorganismen in einer vorgebbaren Zeit benötigt wird, um in Abwasser enthaltene organische Stoffe bei einer bestimmten Temperatur (in der Regel 20° C) oxidativ abzubauen.

Eine aus der Zeitschrift "GWF-Wasser/Abwasser" (1976), 117, Heft Nr. 10, S. 454 - 461, bekannte Vorrichtung zur Bestimmung des biochemischen Sauerstoffbedarfs umfaßt einen Reaktionsbehälter, einen Sauerstofferzeuger und einen Druckindikator als jeweils selbständige Baueinheiten, die durch Polyvinylchlorid-Schläuche miteinander zu verbinden sind.

Das dabei angestrebte druckdicht geschlossene Meßsystem ist unabhängig von barometrischen Luftdruckschwankungen. Die zu untersuchende Probe wird dabei im Reaktionsbehälter durch ein Rührwerk umgewälzt, wobei die zum Abbau der organischen Substanzen erforderliche Sauerstoffmenge aus dem Gasraum des Reaktionsbehälters in das Substrat eindringt. Bei den Kohlenstoffwechselvorgängen der Mikroorganismen entsteht Kohlendioxid, das in den Gasraum diffundiert und gebunden wird. Aufgrund der Kohlendioxid-Absorption fällt ein Unterdruck an, auf den der Druckindikator anspricht und über einen Schaltverstärker die Sauerstofferzeugung bis zum Druckausgleich in Tätigkeit setzt.

Nachteilig bei der bekannten Vorrichtung ist, daß diese aufgrund der Notwendigkeit einer Vielzahl von Einzelvorrichtungskomponenten, die in einem sogenannten Brutschrank zusammenzufassen sind, außerordentlich aufwendig ist. Demzufolge sind Bauaufwand und Platzbedarf erheblich. Stark verschmutzte Abwasserproben lassen sich - nicht zuletzt aufgrund der beschränkten Meßkolbengröße - nur bedingt untersuchen, wobei allerdings eine vorherige Verdünnung notwendig ist. Dessenungeachtet sind exakte Messungen von sehr hohen und sehr niedrigen BSB-Werten nur in sehr unzulänglicher Weise möglich, da aufgrund von systembedingten Druckundichtigkeiten Meßergebnisse verfälscht werden.

Aus der US-PS 3,668,102 ist darüber hinaus eine Vorrichtung der eingangs genannten Art bekannt, bei der auf den die Probenflüssigkeit aufnehmenden Reaktionsbehälter zunächst ein Verbindungsstück aufsetzbar ist, das zur Halterung eines von oben in diesen einsetzbaren Elektrolysegerätes dient. Das Elektrolysegerät erstreckt sich domartig nach oben hin und hat zwei ringförmige Kammern zur Aufnahme der Elektrolyseflüssigkeit. Diese Kammern stehen über Bohrungen in Verbindung und sind gleichfalls über Öffnungen mit der Atmosphäre sowie über nutenförmige Öffnungen mit dem Gasraum des Reaktionsbehälters zu verbinden. Aufgrund der erheblichen Anzahl von vorzusehenden Einzelbauteilen ist auch diese Vorrichtung aufwendig und aufgrund nahezu unvermeidbarer Undichtigkeiten nicht in der Lage, bei stark verschmutzten Abwasserproben exakte Messungen von sehr hohen und sehr niedrigen BSB-Werten durchzuführen. Dies führt im Regelfall dazu, daß insbesondere länger andauernde Messungen nur atmosphärendruckabhängig möglich sind, so daß Meßergebnisse verfälscht werden. Im übrigen sind die hier vorgesehenen Elektroden nur über eine besondere Schaltvorrichtung zu aktivieren, womit ein zusätzlicher Bau- und Bedienaufwand mit weiterer Gefahr von Meßergebnisverfälschungen einhergeht.

Es ist daher Aufgabe der vorliegenden Erfindung, eine Vorrichtung der eingangs genannten Art zu schaffen, die in baulich einfacher Weise mit verringertem Platzbedarf auch die exakte Messung stark verunreinigter Abwässer im Alltagsbetrieb zuläßt.

Zur Lösung dieser Aufgabe zeichnet sich die Vorrichtung der eingangs genannten Art durch die im kennzeichnenden Teil des Anspruchs 1 angegebenen Merkmale aus. Hinsichtlich wesentlicher weiterer Ausgestaltungen der Erfindung wird auf die Ansprüche 2 bis 9 verwiesen.

Mit der Vorrichtung nach der Erfindung lassen sich in handhabungstechnisch einfacher Weise ohne das Erfordernis einer vorherigen Verdünnung stark verschmutzte Abwasserproben und somit auch sehr hohe und sehr niedrige BSB-Werte durch einfache Variation des Probenvolumens direkt messen. Aufgrund der Zusammenfassung von Reaktionsbehälter und Elektrolysegerät ist zudem die Dichtigkeit des Systems in erheblicher Weise verbessert, so daß Meßwertverfälschungen nahezu ausgeschlossen sind. Zudem baut die Vorrichtung außerordentlich kompakt und erfordert damit einen gegenüber herkömmlichen Vorrichtungen weit verringerten Bauraum.

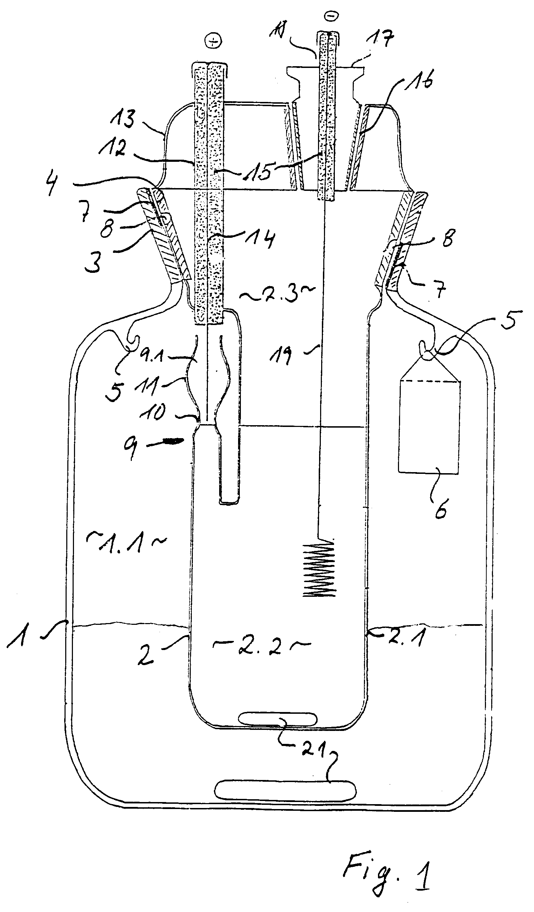

Hinsichtlich weiterer Merkmale und Vorteile der Erfindung wird auf die Zeichnung sowie die nachfolgende Beschreibung verwiesen. In der einzigen Figur der Zeichnung ist ein Ausführungsbeispiel in einer schematischen Querschnittsdarstellung näher veranschaulicht.

Das in der Zeichnung veranschaulichte Ausführungsbeispiel der Vorrichtung zur Bestimmung des biologischen Sauerstoffbedarfes in fünf Tagen (BSB 5) umfaßt einen allgemein mit 1 bezifferten Reaktionsbehälter, der in dem Ausführungsbeispiel durch eine zylindrische Glasflasche mit Flachboden und angesetztem Innenschliff 3 gebildet ist. Bei dem dargestellten Ausführungsbeispiel ist der Innenschliff 3 ca. 3,5 cm hoch und weist an seiner engsten Stelle einen Innendurchmesser von etwa 7 cm auf. Der Innendurchmesser des Reaktionsbehälters liegt bei etwa 14 cm, wobei das Volumen ca. 2 l beträgt.

In den Reaktionsbehälter 1 ist ein allgemein mit 2 beziffertes Elektrolysegerät einhängbar, das ebenfalls aus Glas besteht und einen oberen Außenschliff 4 hat, der an dem Innenschliff 3 des Reaktionsbehälters 1 anzulegen ist. Das Elektrolysegerät 2 kann somit in dem Innenraum des Reaktionsbehälters 1 eingesetzt werden, wobei über die dichtenden, vom Innenschliff 3 sowie vom Außenschliff 4 gebildeten Kontaktflächen die angestrebte Dichtheit des Systems gewährleistet wird.

An der Innenwandung des Reaktionsbehälters sind Halter 5 ausgebildet, an die CO₂-Absorber anzuhängen sind. Der Innenschliff 3 des Reaktionsbehälters umfaßt zudem zwei eingeschliffene Nuten 7, die mit zwei im Außenschliff 4 des Elektrolysegerätes 3 vorgesehene Bohrungen 8 derart zusammenwirken, daß durch Verdrehen des Elektrolysegerätes der Reaktionsraum 1.1 des Reaktionsbehälters 1 belüftet werden kann.

Das Elektrolysegerät 2 hat unterhalb des Außenschliffes 4 ein zylindrisches Gefäßteil 2.1, an den sich ein rohrförmiges Steigrohr 9 anschließt. Um durch das Steigrohr 9 das Elektrolysegerät nicht zu verbreitern, ist dieses im Bereich seiner Erstreckung mit einem geringeren Durchmesser ausgebildet. Das Steigrohr 9 hat eine Verengung 10, an die sich eine im Querschnitt olivenförmige Erweiterung 11 anschließt. Das Steigrohr 9 ist zum Reaktionsraum 1.1 des Reaktionsbehälters hin offen.

Oberhalb des Steigrohres 9 mündet in den Reaktionsraum 1.1 ein weiteres Glasrohr 12, das durch den oberen Deckel 13 des Elektrolysegerätes 2 nach außen geführt ist und zur Aufnahme einer ca. 0,6 mm dicken Platinelektrode 14 dient, die zentriert bis in etwa zur Verengung 10 des Steigrohres 9 des Elektrolysegerätes 2 reicht. Die Platinelektrode ist innerhalb des Glasrohres 12 mit Silikonkautschuk abgedichtet gehaltert.

Der Deckel 13 des Elektrolysegerätes 2 hat eine Bohrung 16 mit Innenschliff, durch die das Elektrolysegerät mit einer allgemein mit 2.2 bezifferten Elektrolyseflüssigkeit gefüllt werden kann. Zum Verschließen der Bohrung 16 ist ein Glasstopfen 17 mit Außenschliff vorgesehen, durch den in analoger Weise wie bei der Platinelektrode 14 ein weiteres Glasrohr 18 geführt ist, das eine Kupferelektrode 19 haltert. Die Kupferelektrode 19 ist gleichfalls mit Silikonkautschuk 15 verpreßt. Die Platinelektrode 14 und die Kupferelektrode 19 ragen nach außen, so daß an diese Stecker für die Verbindung mit der nicht näher dargestellten Steuer- und Meßwertverarbeitungseinrichtung angeschlossen werden können. Sowohl im Elektrolysegerät 2 als auch im Reaktionsraum 1.1 sind Rührer 21 vorgesehen.

Im Betrieb wird in den Reaktionsraum 1.1 des Reaktionsbehälters die zu untersuchende Abwasserprobe eingegeben. Je nach Erfordernis und Verschmutzungsgrad kann dabei das Probenvolumen variiert werden und z. B. bis zu 1000 ml betragen. Nachdem die CO₂-Absorber 6 in den Reaktionsbehälter eingehängt sind, wird die Probe mit einem langen Trichter eingefüllt und der Rührer 21 (Rührfisch) und eine gewisse Menge an Mikroorganismen hinzugegeben. Hiernach wird das Elektrolysegerät 2 in den Reaktionsbehälter 1 eingesetzt, wonach dieser verschlossen ist. Das Elektrolysegerät 2 wird über die Bohrung 16 mit einer gesättigten CuSO₄-Lösung, einigen Gramm CuSO₄ und einigen Tropfen H₂SO₄ gefüllt. Die Höhe des Flüssigkeitsspiegels der CuSO₄-Lösung wird dabei so eingestellt, daß dieser in der Verengung 10 des Steigers 9 bis ca. 1 mm unterhalb des Endes der Platinelektrode 14 gelegen ist. Ein Rührfisch 21 wird gleichfalls in das Elektrolysegerät 2 gegeben und dieses mit dem die Kupferelektrode 19 tragenden Glasstopfen 17 verschlossen. Zum Druckausgleich zwischen Atmosphäre, Luftraum des Reaktionsbehälters 1 und Luftraum des Elektrolysegerätes 2 ist danach das Elektrolysegerät derart zu drehen, daß die Nuten 7 im Innenschliff des Reaktionsbehälters mit den Bohrungen 8 im Außenschliff des Elektrolysegerätes zur Deckung kommen. Als Ausgangswert stellt sich somit Atmosphärendruck in der gesamten Geräteeinheit sein. Durch eine abermalige Verdrehung des Elektrolysegerätes um ca. 90° ist die Geräteeinheit abgedichtet und der Reaktionsraum 1.1 druckmäßig von dem Luftraum des Elektrolysegerätes 2 getrennt. Jede Druckschwankung im Luftraum des Reaktionsbehälters wird sich jetzt in Schwankungen des Flüssigkeitsspiegels im Steigrohr 9 niederschlagen. Die so vorbereitete Geräteeinheit kann in einen Thermostaten bei ca. 21°C gebracht werden. Die Probenlösung für die Kupfersulfatlösung wird dabei koninuierlich durch die Röhre 21 umgewälzt.

Bei der danach einsetzenden Sauerstoffzehrung durch die eingegebenen Mikroorganismen in der Probenlösung kommt es im Reaktionsbehälter 1 zu einem Druckabfall, der ein Ansteigen der CuSO₄-Lösung im Steigrohr 9 bewirkt. Bei Kontakt der CuSO₄-Lösung mit der unter 14-V-Gleichspannung stehenden Spitze der Platinelektrode 14 setzt die Elektrolyse ein. An der positiv geladenen Platinelektrode wird hierbei Sauerstoff produziert, der aus der Öffnung des Steigrohres 9 in den Luftraum des Reaktionsbehälters 1 entweicht, wobei der Druckausgleich wieder hergestellt wird. Bei Druckausgleich reißt der Kontakt zwischen CuSO₄-Lösung und Platinelektrode ab und die Elektrolyse stoppt. An der negativ geladenen Kupferelektrode 19 scheidet sich bei dem Elektrolysevorgang Kupfer ab. Von den Mikroorganismen gebildetes CO₂ wird von den CO₂-Absorbern 6 absorbiert und trägt somit nicht zum Druckausgleich im Reaktionsbehälter bei. Wird von den Mikroorganismen kein Sauerstoff mehr verbraucht, kommt die Elektrolyse endgültig zum Stillstand.

Über die Pole der Elektroden 14 und 19 wird die Einheit mit Gleichstrom gespeist. Zwischen Gleichstromquelle und Geräteeinheit befindet sich eine elektrische Schaltung, in der es an einem definierten Widerstand bei Einsetzen der Elektrolyse zu einem Spannungsabfall kommt. Dieser bei jedem Einschalten der Elektrolyse auftretende Spannungsabfall wird registriert und kann in einem 8 BitAnalog/Digitalwandler in digitale Signale umgewandelt werden, die in einem Mikrocomputer zu addieren sind. Über ein entsprechendes Programm läßt sich so der in der gesamten Zeit bei der Elektrolyse geflossene Strom und damit direkt der neugebildete Sauerstoff berechnen. Dieser entspricht der Menge Sauerstoff, die von Mikroorganismen verbraucht wurde.

## Patentansprüche

1. Vorrichtung zur Bestimmung des biochemischen Sauerstoffbedarfs und der Toxizität von insbesondere Abwässern mit einen einen Reaktionsraum (1.1) zur Aufnahme einer Flüssigkeitsprobe umfassenden Reaktionsbehälter (1), einem Elektrolysegerät (2), einem in dem Reaktionsbehälter (1) vorsehbaren CO₂-Absorber (6) sowie einer mit dem Elektrolysegerät (2) verbindbaren Steuer- und Meßwertverarbeitungseinrichtung, wobei der Reaktionsbehälter (1) und das Elektrolysegerät (2) eine trennbare Baueinheit bilden, der Reaktionsbehälter über das Elektrolysegerät (2) verschließbar ist, das Elektrolysegerät (2) in Strömungsverbindung stehende Kammern hat, denen jeweils eine Elektrode (14,19) zugeordnet ist und eine der Kammern zum Gasraum des Reaktionsbehälters (1) hin offen ist, **dadurch gekennzeichnet,** daß das mit einer Strommeßeinrichtung verbundene Elektrolysegerät (2) in den Innenraum des Reaktionsbehälters einsetzbar ist und daß das Elektrolysegerät (2) ein eine der Kammern (14,19) bildendes Gefäßteil (2.1) umfaßt, an das sich ein in den freien Reaktionsgasraum mündendes, die andere Kammer bildendes Steigrohr (9) anschließt, wobei eine der Elektroden (14) bis in das Steigrohr (9) ragt.

2. Vorrichtung nach Anspruch 1, **dadurch gekennzeichnet,** daß das Elektrolysegerät (2) in den Reaktionsbehälter (1) einhängbar ist.

3. Vorrichtung nach Anspruch 1 oder 2, **dadurch gekennzeichnet****,** daß in den Kontaktflächen (3, 4) des Elektrolysegerätes (2) und des Reaktionsbehälters (1) Nuten (7) und Bohrung (8) zur Belüftung des Reaktionsraumes (1.1) des Reaktionsbehälters (1) vorgesehen sind.

4. Vorrichtung nach einem der Ansprüche 1 bis 3, **dadurch gekennzeichnet****,** daß das Steigrohr (9) Teile mit verringerten Querschnittsabmessungen aufweist.

5. Vorrichtung nach einem der Ansprüche 1 bis 4, **dadurch gekennzeichnet****,** daß das Steigrohr (9) eine Querschnittsverengung (10) sowie sich eine daran anschließende Querschnittsverbreiterung (11) aufweist.

6. Vorrichtung nach Anspruch 5, **dadurch gekennzeichnet****,** daß eine der Elektrode (14) bis hin zur Querschnittsverengung (10) in das Steigrohr (9) ragt.

7. Vorrichtung nach einem der Ansprüche 1 bis 6, **dadurch gekennzeichnet****,** daß das Elektrolysegerät (2) eine durch einen Stopfen (17) verschließbare Füllöffnung (16) hat, wobei an dem Stopfen (17) eine der Elektroden (19) gehaltert ist.

8. Vorrichtung nach einem der Ansprüche 1 bis 7, **dadurch gekennzeichnet****,** daß an der Innenwandung des Reaktionsbehälters Halter (5) zum Anhängen eines Absorbers (6) vorgesehen sind.

9. Vorrichtung nach einem der Ansprüche 1 bis 8, **dadurch gekennzeichnet****,** daß die im Steigrohr (9) befindliche Elektrode (14) nach Befüllung mit Probenflüssigkeit zu Beginn eines Meßvorganges oberhalb des Elektrolyseflüssigkeitsspiegels endet.

## Claims

1. A device for determining the biochemical oxygen requirement and toxicity of waste water in particular, with a reaction tank (1) comprising a reaction container (1.1) adapted to accommodate a sample of liquid, and further comprising an electrolysis unit (2), a CO₂ absorber (6) which can be provided in the reaction container (1) and also with a controlling and measured value processing means adapted to be connected to the electrolysis unit (2), the reaction container (1) and the electrolysis unit (2) forming one separable component, the reaction container being adapted to be occluded via the electrolysis unit (2), the electrolysis unit (2) having in the flow connection chambers with each of which an electrode (14, 19) is associated and in that one of the chambers is open towards the gas space in the reaction container (1), characterised in that the electrolysis unit (2) connected with a flow measuring means can be inserted into the interior of the reaction container and in that the electrolysis unit (2) comprises, constituting one of the chambers (14, 19), a vessel part (2.1) adjacent to which there is, discharging into the free reaction gas space a riser pipe (9) which forms the other chamber, one of the electrodes (14) projecting into the riser pipe (9).

2. A device according to claim 1, characterised in that the electrolysis unit (2) can be suspended in the reaction container (1).

3. A device according to claim 1 or 2, characterised in that grooves (7) and bores (8) for ventilating the reaction chamber (1.1) of the reaction container (1) are provided in the contact surfaces (3, 4) of the electrolysis unit (2) and the reaction container (1).

4. A device according to one of claims 1 to 3, characterised in that the riser pipe (9) comprises parts of reduced cross-section.

5. A device according to one of claims 1 to 4, characterised in that the riser pipe (9) comprises a cross-sectional narrowing (10) and, adjacent thereto, a cross-sectional widening (11).

6. A device according to claim 5, characterised in that one of the electrodes (14) projects into the riser pipe (9) as far as the cross-sectional narrowing (10).

7. A device according to one of claims 1 to 6, characterised in that the electrolysis unit (2) has a filling aperture (16) adapted to be occluded by a plug (17), one of the electrodes (19) being supported on the plug (17).

8. A device according to one of claims 1 to 7, characterised in that holders (5) are provided on the inside walls of the reaction container for the attachment of an absorber (6).

9. A device according to one of claims 1 to 8, characterised in that after being filled with test liquid at the commencement of a measuring process, the electrode (14) disposed in the riser pipe (9) ends above the surface of the electrolysis liquid.

## Revendications

1. Appareil pour déterminer le besoin biochimique en oxygène et la toxicité d'eaux usées en particulier, avec un récipient de réaction (1) comprenant une chambre de réaction (1.1) pour la réception d'un échantillon liquide, un appareil d'électrolyse (2), un absorbeur de CO₂ (6) prévu dans le récipient de réaction (1) ainsi qu'un dispositif pour le traitement de valeurs de conduite et de mesure, susceptible d'être relié à l'appareil d'électrolyse 2, dans lequel le récipient de réaction (1) et l'appareil d'électrolyse (2) forment une unité de construction susceptible d'être séparée, le récipient de réaction est obturable par l'appareil d'électrolyse (2), l'appareil d'électrolyse (2) a des chambres reliées par une liaison dynamique, chacune comprend une électrode (14, 19) et une des chambres est ouverte vers la chambre à gaz du récipient de réaction (1), caractérisé en ce que l'appareil d'électrolyse (2), relié à un ampèremètre, est susceptible d'être introduit dans la chambre interne du récipient de réaction, et que l'appareil d'électrolyse (2) comprend un vase (2.1) engendrant une des chambres (14, 19), à partir de quoi le tube ascendant (9) constituant à l'autre chambre débouche librement dans la chambre à gaz de réaction, où une des électrodes (14) dépasse jusque dans ce tube ascendant (9).

2. Appareil suivant la revendication 1, caractérisé en ce que l'appareil d'électrolyse (2) est susceptible d'être suspendu dans le récipient de réaction (1).

3. Appareil suivant la revendication 1 ou 2, caractérisé en ce que des rainures (7) et des orifices (8) sont prévus aux surfaces de contact (3, 4) de l'appareil d'électrolyse (2) et du récipient de réaction (1) pour l'aération de la chambre de réaction (1.1) du récipient de réaction (1).

4. Appareil suivant les revendications 1 à 3, caractérisé en ce que le tube ascendant (9) présente une partie dont la mesure de section transversale est diminuée.

5. Appareil suivant les revendications 1 à 4, caractérisé en ce que le tube ascendant (9) présente un rétrécissement de section transversale (10), ainsi qu'un élargissement de section transversale (11) y adapté.

6. Appareil suivant la revendication 5, caractérisé en ce qu'une des électrodes (14) dépasse jusqu'à l'élargissement de section transversale (10) du tube ascendant (9).

7. Appareil suivant les revendications 1 à 6, caractérisé en ce que l'appareil d'électrolyse (2) a une ouverture de remplissage (16), obturable par un bouchon (17), où une des électrodes (19) est fixée au bouchon (17).

8. Appareil suivant les revendications 1 à 7, caractérisé en ce qu'un support (5) est prévu dans la paroi interne du récipient de réaction pour suspendre un absorbeur (6).

9. Appareil suivant les revendications 1 à 8, caractérisé en ce que l'électrode (14) se trouvant dans le tube ascendant (9) aboutit au-dessus du niveau de liquide d'électrolyse, après remplissage avec le liquide d'échantillon pour commencer un processus de mesure.
